(19) Europäisches Patentamt
European Patent Office
Office européen des brevets

(11) EP 1 008 580 B2

(12) **NEW EUROPEAN PATENT SPECIFICATION**
After opposition procedure

(45) Date of publication and mention of the opposition decision:
**29.10.2008 Bulletin 2008/44**

(45) Mention of the grant of the patent:
**26.02.2003 Bulletin 2003/09**

(21) Application number: **99124566.3**

(22) Date of filing: **09.12.1999**

(51) Int Cl.:
***C07C 45/50*** *(2006.01)* ***C07C 29/141*** *(2006.01)*

# (54) Process for preparing aldehydes and alcohols

Verfahren zur Herstellung von Aldehyden und Alkoholen

Procédé pour la préparation d' aldéhydes et d' alcools

(84) Designated Contracting States:
**DE NL**

(30) Priority: **10.12.1998 JP 35111598**

(43) Date of publication of application:
**14.06.2000 Bulletin 2000/24**

(73) Proprietor: **Mitsubishi Chemical Corporation**
**Minato-ku**
**Tokyo 108-0014 (JP)**

(72) Inventors:
- **Takagi, Masatoshi,**
**c/o Mitsubishi Chemical Corp.**
**Yokohama-shi,**
**Kanagawa (JP)**
- **Urata, Hisao,**
**c/o Mitsubishi Chemical Corp.**
**Yokohama-shi,**
**Kanagawa (JP)**

(74) Representative: **ter Meer, Nicolaus**
**TER MEER STEINMEISTER & PARTNER GbR,**
**Patentanwälte,**
**Mauerkircherstrasse 45**
**81679 München (DE)**

(56) References cited:
**US-A- 4 533 755**
**US-A- 4 716 250**
**US-A- 5 189 105**
**US-A- 5 808 168**

Remarks:
The file contains technical information submitted after the application was filed and not included in this specification



Printed by Jouve, 75001 PARIS (FR)

## Description

The present invention relates to a process for preparing aldehydes and alcohols, and particularly relates to a hydroformylation reaction system to obtain aldehydes at a high activity and a high selectivity.

It is well known to hydroformylate an olefinic unsaturated compound with a $H_2$/CO mixed gas in the presence of a catalyst and it is also known to use a continuous multistage flow reactor as a method for industrially favorably conducting this reaction.

For example, JP-A-61-218546 discloses to use a two stage decouple reaction system wherein a gaseous distillate containing unreacted olefin, $H_2$ and CO remained after reaction in the first stage reactor is supplied as it is to the second stage reactor to carry out hydroformylation reaction, and also discloses to operate by using a separate catalyst solution-withdrawing step and a separate product-withdrawing step to each reactor. This reference discloses that a conversion of an olefin to an aldehyde is improved and also flexibility of the operation is improved, as effects of the decouple reaction system.

Also, JP-A-3-204831 discloses a method for hydroformylation of an olefin by using a specific combination of reaction systems having two different mixing properties, and discloses a method for obtaining an aldehyde at a high yield by preventing formation of by-products such as a low boiling point material, a high boiling point material and the like.

US-A-4,716,250 relates to a rhodium-monosulfonated tertiary phosphine salt ligand complex catalyzing the reaction of olefinic compounds to their corresponding aldehydes in a non-aqueous hydroformylation reaction.

However, although these prior arts provide a method for obtaining an aldehyde at a high yield by raising an olefin conversion, there was no prior art providing a method satisfying both of a high olefin conversion and a high selectivity of a product, which are industrially important performances. Heretofore, it has been considered that a high olefin conversion and a high selectivity of a product are performances opposed to each other, and it has been technically difficult to provide a process satisfying both of the opposed two performances.

The present inventors have studied a process for hydroformylation of an olefinic unsaturated compound by using a continuous multistage reactor, and have noted that a pressure relation between each reactor has an influence on a reaction activity and a selectivity. As this result, the present inventors have discovered that a product can be obtained at a higher reaction rate and a higher selectivity by carrying out hydroformylation reaction by dividing into a plurality of pressure sections by using a multistage reactor, as compared with an operation under a single reaction pressure, and the present invention has been accomplished on the basis of this discovery.

That is, the essential feature of the present invention resides in a process for preparing aldehydes which comprises reacting an olefinic unsaturated compound with hydrogen and carbon monoxide by hydroformylation reaction in the presence of a catalyst in a continuous multistage flow reactor, wherein the continuous multistage flow reactor contains n number ($n \geqq 2$) of flow reactors, and the reaction is carried out in the presence of pressure sectional zones satisfying at least pressure condition (2) of the following pressure conditions (1) to (3), provided that partial pressures of hydrogen and carbon monoxide in each reactor are illustrated as ($P_{H2}(1)$,$P_{CO}(1)$), ($P_{H2}(2)$,$P_{CO}(2)$), ··· ($P_{H2}(n)$,$P_{CO}(n)$) in order from the upper stream from the reactor,

$$(1) \qquad P_{CO}(m-1)<P_{CO}(m)$$

$$(2) \qquad P_{H2}(m-1)<P_{H2}(m)$$

$$(3) \qquad P_{CO}(m-1)+P_{H2}(m-1)<P_{CO}(m)+P_{H2}(m)$$

in which m is an integer in the range of $2 \leqq m \leqq n$.

Hereinafter, the present invention is further described in more details.

The present invention provides a process for preparing aldehydes by hydroformylating an olefinic unsaturated compound with hydrogen and carbon monoxide in the presence of a catalyst in a continuous multistage flow reactor.

The continuous multistage flow reactor used in the present invention is a reactor obtained by connecting a plurality of reactors in series. Usually, the number (n) of reactors is from 2 to 10, preferably from 2 to 5, particularly preferably 2, but the number of reactors can be determined optionally depending on reaction conditions. The reactors used herein may be any type of reactors so long as mixing of gas-liquid can be satisfactorily carried out, and examples include a stirring tank type reactor, a plug flow type reactor and the like. Respective reactors in the continuous multistage

reactor may be the same type, or plural types of reactors may be combined.

Operation conditions of the continuous multistage reactor used in the present invention vary substantially depending on a kind of an olefinic unsaturated compound used, but are generally as illustrated below. A reaction temperature is from 20 to 200°C, preferably from 50 to 160°C, more preferably from 100 to 150°C. The reaction temperature of each reactor may be the same or different. It is preferable that a total pressure of $H_2$/CO mixed gas used is in the range of from 10 to 300 kg/cm$^2$, preferably from 30 to 250 kg/cm$^2$, in all of reactors.

As mentioned above, the present invention employs a n number ($2 \leqq n$) of continuous flow reactors, and is characterized by achieving a high olefin conversion, a high selectivity, e.g. a high n/i ratio and a high terminal formylation rate by controlling a hydrogen partial pressure, a carbon monoxide partial pressure or their total pressure.

Particularly, the reaction is carried out in the presence of pressure sectional zones satisfying at least pressure condition (2) of the following pressure conditions (1) to (3), provided that partial pressures of hydrogen and carbon monoxide in each reactor are illustrated as ($P_{H2}(1)$,$P_{CO}(1)$), ($P_{H2}(2)$,$P_{CO}(2)$), ··· ($P_{H2}(n)$,$P_{CO}(n)$) in order from the upper steam from the reactor,

$$(1) \qquad P_{CO}(m-1)<P_{CO}(m)$$

$$(2) \qquad P_{H2}(m-1)<P_{H2}(m)$$

$$(3) \qquad P_{CO}(m-1)+P_{H2}(m-1)<P_{CO}(m)+P_{H2}(m)$$

in which m is an integer in the range of $2 \leqq m \leqq n$.

In the present invention, at least two reactors connected in series in the continuous multistage flow reactor have only to satisfy the above conditions, and pressure conditions in other reactors are not restricted. Particularly, it is preferable to satisfy the condition of m=2 in any one of the formulae (1) to (3).

By providing pressure sectional zones satisfying at least pressure condition (2) of the above formulae (1) to (3), a selectivity (n/i ratio, terminal formylation rate or the like) is preferentially raised in a (m-1) stage reactor having a relatively low pressure, and it is possible to raise a conversion of an olefinic compound in a m stage reactor having a relatively high pressure.

Also, more preferably, it is favorable to employ pressure sectional zones satisfying the following conditions.

$$P_{CO}(m)/P_{CO}(m-1) = 1.5\text{-}5$$

$$PH_2(m)/P_{H2}(m-1) = 2\text{-}5$$

$$(P_{CO}(m)+P_{H2}(m))/(P_{CO}(m-1)+P_{H2}(m-1)) = 1.5\text{-}5$$

More particularly, when among hydrogen partial pressures of n number of reactors, the lowest value is A and the highest value is B, the reaction is carried out in pressure sectional zones of B/A = 1.2-10, preferably 1.3-8, more preferably 1.5-5, or when among carbon monoxide partial pressures of n number of reactors, the lowest value is C and the highest value is D, it is preferable to carry out the reaction in pressure sectional zones D/C = 1.8-10, preferably 1.9-8, more preferably 2-5. Among them, it is more preferable to carry out the reaction under such conditions as satisfying both of B/A and D/C in the above-mentioned ranges, and more particularly, when among a total pressure of hydrogen and carbon monoxide in n number of reactors, the lowest value is E and the highest value is F, it is preferable to carry out the reaction in pressure sectional zones of F/E = 1.1-10, preferably 1.3-8, more preferably 1.5-5.

A $H_2$/CO ratio in a $H_2$/CO mixed gas used is not specially limited, but is usually 1/5-5/1, preferably 1/3-3/1, more preferably 1/2-2/1. It is most preferable to use a mixed gas having a $H_2$/CO ratio of about 1/1 which is industrially

easily available. The "partial pressure" defined in the present invention means a partial pressure of a gas phase of a reactor in the case of a stirring tank type reactor, and means an average partial pressure of a partial pressure at the inlet and a partial pressure at the outlet of a reactor in the case of a reactor having a pressure distribution in the reactor such as a plug flow type reactor.

When carrying out the reaction, a reaction starting material of liquid phase is transferred from the upper reactor to the lower reactor, but with regard to a reaction starting material of gas phase, $H_2$/CO gas may be supplied independently to each reactor, and a gas containing $H_2$/CO gas may be withdrawn from a gas phase of one reactor and may be supplied to another reactor as a supplying gas.

In the reaction system of the present invention, there may be employed a stripping system which comprises continuously supplying an unsaturated compound and an oxo gas to a reaction zone having a liquid phase containing a rhodium complex catalyst and having the formed aldehyde together with an unreacted oxo gas withdrawn from the reaction zone or there may be employed a liquid cycling system which comprises continuously supplying a reaction solvent containing a catalyst, an unsaturated compound and an oxo gas to a reaction zone, continuously withdrawing a reaction product solution containing the formed aldehyde from the reaction zone, separating at least a part of the formed aldehyde, and recycling a reaction solvent containing a remaining catalyst to the reaction zone.

An average residence time in each reactor constituting the continuous multistage flow reactor is fixed so as to achieve an aimed reaction conversion under practical reaction conditions, and is usually from 1 minute to 10 hours, preferably from 10 minutes to 8 hours, more preferably from 30 minutes to 7 hours.

Depending on operation conditions of the reactor, an aldehyde product is further hydrogenated to produce an alcohol at the same time. Accordingly, when an aimed final product is an alcohol, a load of hydrogenation step at a later stage can be omitted or reduced. By employing the process of the present invention, it is possible to increase an amount of an alcohol produced together with an aldehyde, and an industrial merit is great in respect that a load of hydrogenation step at a later stage can be reduced.

An olefinic unsaturated compound used in the present invention is an unsaturated organic compound having at least one carbon-carbon double bond, some of which have a functional group and others of which have no functional group. Examples of having no functional group include $C_3$-$C_{12}$, preferably $C_4$-$C_{10}$, unsaturated compounds and their isomers, such as propene, butenes, butadienes, pentenes, pentadienes, hexenes, octenes, nonenes, decenes and the like. Also, examples of having a functional group include allyl alcohol, allyl ethers, acrolein, acrylates, butenals, butenic acid esters, pentenals, pentenic acid esters, and the like. In the present invention, any of an internal olefin and a terminal olefin can be used, but an internal olefin is preferable in respect that an effect for improving a selectivity of a terminal isomerized product industrially demanded can be fully achieved. Also, it is preferable to employ a plurality of mixtures of these olefins, and it is particularly preferable to employ mixed octene obtained by dimerization of butene which is industrially easily available.

A reaction conversion of an olefinic unsaturated compound is usually 50-100%. Particularly, when the olefinic unsaturated compound is a mixture of at least two kinds of compounds, it is preferable to make a reaction conversion high, at least 75%, preferably at least 80%, more preferably at least 85%.

A catalyst used in the present invention may be any catalyst so long as it is effective for hydroformylation of an olefinic unsaturated compound. Examples of a metal component contained in the catalyst include Group 8 to Group 10 elements of the Periodic Table such as rhodium cobalt, ruthenium, palladium, platinum, iron and the like, and among them, rhodium or cobalt is preferable, and rhodium is most preferable.

When using rhodium as a catalyst component, 0 valent or cationic compounds of rhodium can be used, examples of which include Rh(NBD)(acac), Rh(COD)(acac), $Rh(acac)_2$, $[Rh(COD)Cl]_2$, $[Rh(NBD)Cl]_2$, $Rh(CO)_2(acac)$, and rhodium carbonyl clusters such as $Rh_4(CO)_{12}$ and $Rh_6(CO)_{i6}$.

It is preferable to employ a metal component in the form of a complex.

Examples of a usable ligand include a compound known in prior arts to be workable as a ligand to a metal used, such as monodentate and multidentate phosphines, phosphites, amines and the like, but it is not always necessary to use such a ligand.

More particular examples of a compound usable as a ligand include phosphines such as a trialkylphosphine and a triarylphosphine including triphenylphosphine, tritolylphosphine or the like.

Examples of phosphites include triarylphosphites, trialkylphosphites, alkylarylphosphites and the like, and optional organic phosphites among the above illustrated phosphites can be used. Also, polyphosphites such as a bisphosphite, a trisphosphite and the like, which have a plurality of these phosphite structures in a molecule, can be used.

Among these phosphites, a monophosphite is classified into one having no cyclic structure containing a phosphorus atom and one having such a structure. The former one is expressed by the following formula (1).

$$P(OR^1)(OR^2)(OR^3) \qquad (1)$$

In the above formula (1), $R^1$ to $R^3$ are respectively independently a $C_1$-$C_{30}$ hydrocarbon group or a $C_5$-$C_{30}$

heteroaromatic hydrocarbon group, such as an alkyl group, a cycloalkyl group, an aryl group, an aralkyl group or the like, and these groups may have a substituent bonded, which does not inhibit hydroformylation reaction. Examples of such a substituent include a halogen atom, a $C_1$-$C_{20}$ alkyl group, a cycloalkyl group, an aryl group, an alkoxy group, an alkylamino group, an acyl group, an acyloxy group, an alkoxycarbonyl group, and the like. Among organic phosphites expressed by formula (1), it is preferable that at least one of $R^1$ to $R^3$ is a substituted aryl group.

Particularly preferable examples of an organic phosphite of the formula (1) include an organic phosphite, all of $R^1$ to $R^3$ of which are substituted aryl groups, particularly a substituted aryl group having a $C_1$-$C_5$ hydrocarbon group as a substituent. Examples of such an organic phosphite include tris(2,4-di-t-butylphenyl)phosphite, tris(2-t-butyl-4-methylphenyl)phosphite, tris(2-t-butyl-4-methoxyphenyl)phosphite, tris(o-phenylphenyl)phosphite, tris(o-methylphenyl) phosphite, bis(3,6-di-t-butyl-2-naphthyl)(2,4-di-t-butylphenyl)phosphite, bis(3,6-di-t-butyl-2-naphthyl)(2-t-butylphenyl) phosphite, tris(3,6-di-t-butyl-2-naphthyl)phosphite, tris(3,6-di-t-amyl-2-naphthyl)phosphite, and the like.

Among monophosphites, one having a cyclic structure containing phosphorus atom is expressed by the following formula (2).

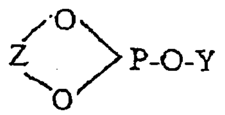

(2)

In the above formula, Z is a divalent hydrocarbon group which may contain a heteroatom in a carbon chain, and Y is a hydrocarbon group or a heteroaromatic hydrocarbon group, which may be substituted.

In the above formula (2), Y is preferably a substituted aryl group. Also, Z is preferably an alkylene group, an arylene group or a mixed group of the two, which may contain a heteroatom such as an oxygen, nitrogen or sulfur atom in a carbon chain. Examples of such a divalent hydrocarbon group include an alkylene group, an alkyleneoxyalkylene group, an alkyleneaminoalkylene group which may have an alkyl group bonded to a nitrogen atom, an alkylenethioalkylene group, a cycloalkylene group, an arylene group, a biarylene group, an alkylenearylene group, an arylenealkylenearylene group, an aryleneoxyarylene group, an aryleneoxyalkylene group, an arylenethioarylene group, an arylenethioarylene group, or an aryleneaminoarylene or aryleneaminoalkylene group which may have an alkyl group bonded to a nitrogen atom, and the like.

Some examples of the above-mentioned organic phosphite having a cyclic structure containing a phosphorus atom are illustrated in the following Table 1.

Table 1

No.1

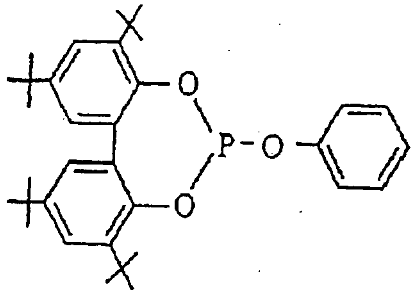

No.2

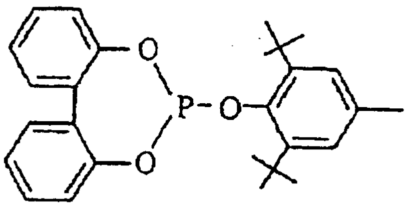

No.3

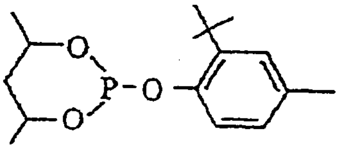

No.4

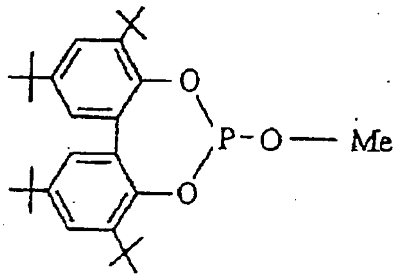

No.5

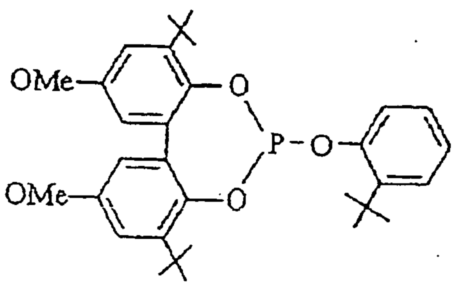

A polyphosphite having at least 2 phosphite structures in a molecule used as a ligand in the present invention is expressed by the following formula (3).

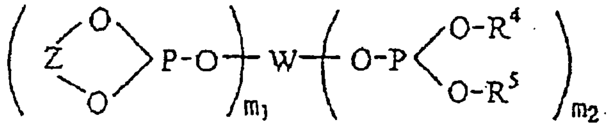

(3)

In the above formula, Z is the same as defined in with regard to the formula (2), and $R^4$ and $R^5$ are respectively independently a $C_1$-$C_{30}$ hydrocarbon group or a $C_5$-$C_{30}$ heteroaromatic hydrocarbon group, such as an alkyl group, a cycloalkyl group, an aryl group, an aralkyl group, and the like, and these groups may have a substituent bonded, which does not inhibit hydroformylation reaction. Examples of such a substituent include a halogen atom, a $C_1$-$C_{20}$ alkyl group, a cycloalkyl group, an aryl group, an alkoxy group, an alkylamino group, an acyl group, an acyloxy group, an alkoxycarbonyl group, and the like.

W is a m-valent hydrocarbon group which may contain a hetero atom such as an oxygen, nitrogen or sulfur atom in a carbon chain. W may further have a substituent bonded. $m_1$ and $m_2$ are respectively a number of from 0 to 6, and $m_1$+$m_2$ is an integer of from 2 to 6. Also, when $m_1$ or $m_2$ is a number of at least 2, a plurality of Z, $R^4$ and $R^5$ may be respectively different.

Preferably, $R^4$ and $R^5$ are an aryl group which may be substituted. Examples of such an aryl group include a phenyl group, a 2-methylphenyl group, a 3-methylphenyl group, a 4-methylphenyl group, a 2,4-dimethylphenyl group, a 2,5-dimethylphenyl group, a 2,6-dimethylphenyl group, a 2-methoxyphenyl group, a 3-methoxyphenyl group, a 4-methoxyphenyl group, a 2,4-dimethoxyphenyl group, a 2,5-dimethoxyphenyl group, a 2,6-dimethoxyphenyl group, an α-naphthyl group, a 3-methyl-α-naphthyl group, a 3,5-dimethyl-α-naphthyl group, a β-naphthyl group, a 1-methyl-β-naphthyl group, a 3-methyl-β-naphthyl group, and the like.

Some examples of the polyphosphite of the formula (3) are illustrated in the following Table 2.

Table 2

No.1

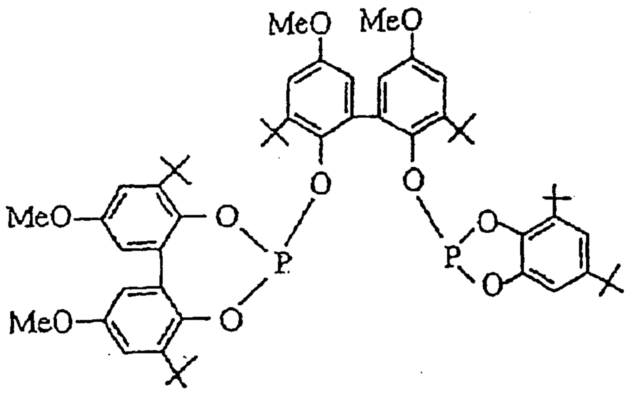

No.2

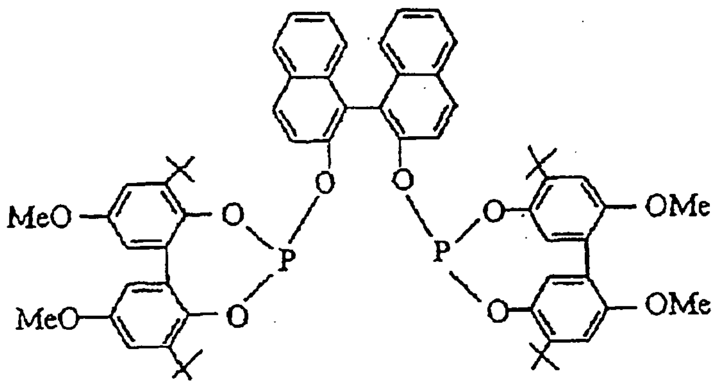

No.3

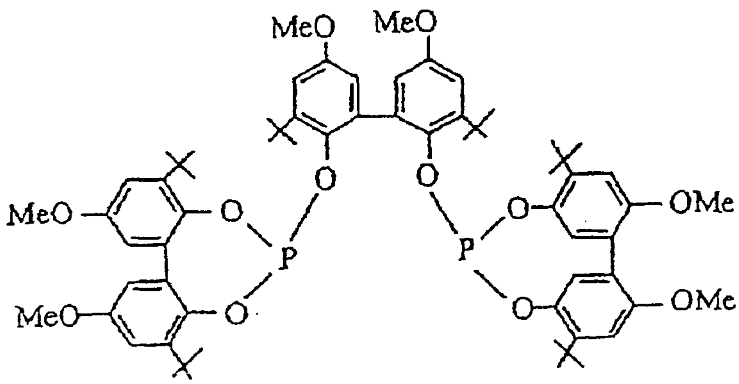

Table 2 (continued)

No.4

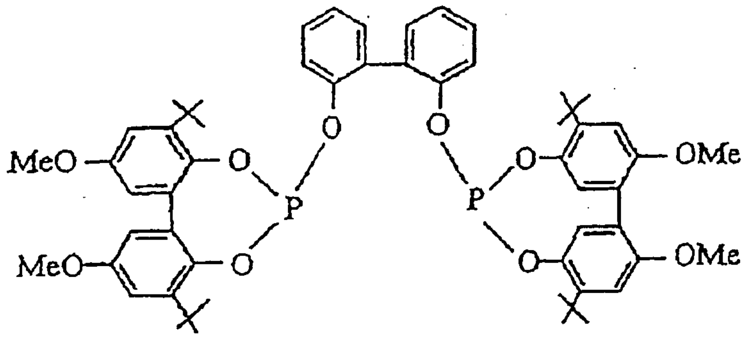

No.5

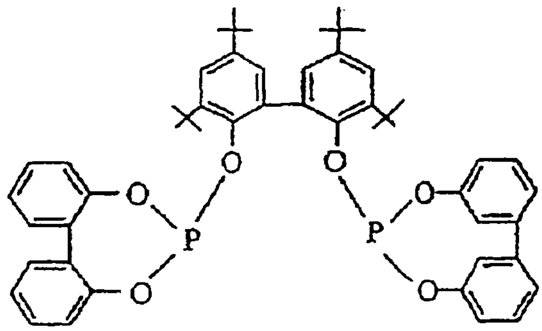

No.6

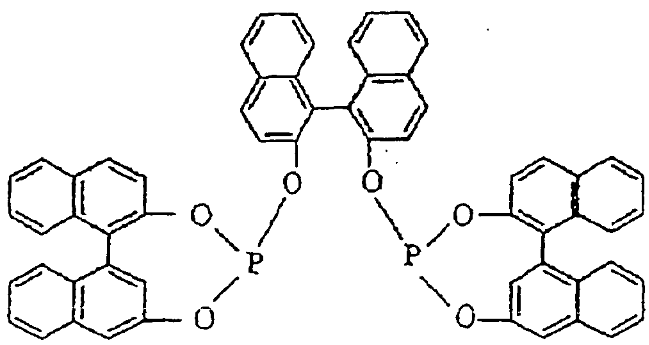

Table 2 (continued)

No.7

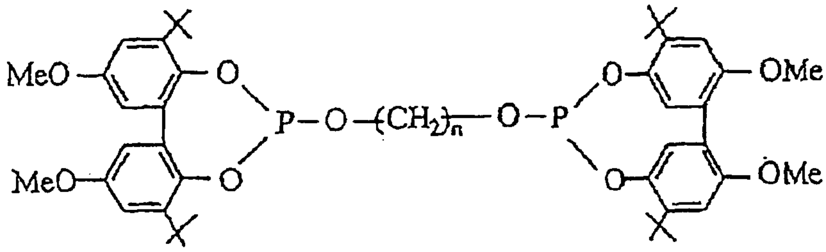

No.8

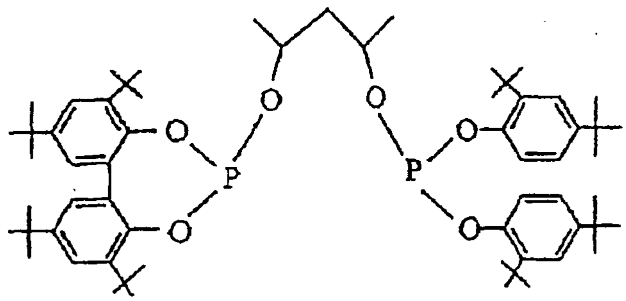

No.9

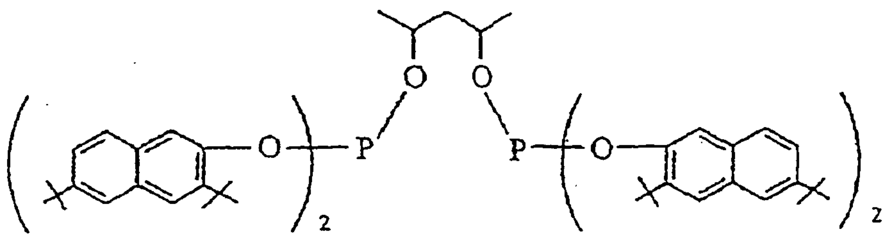

Table 2 (continued)

No.10

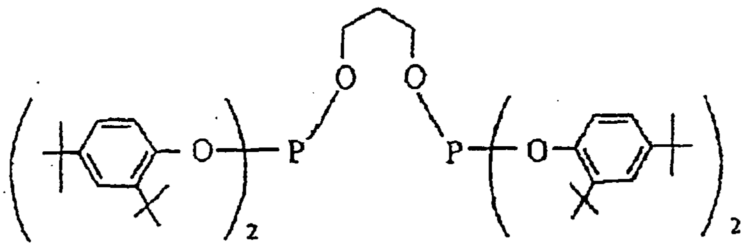

No.11

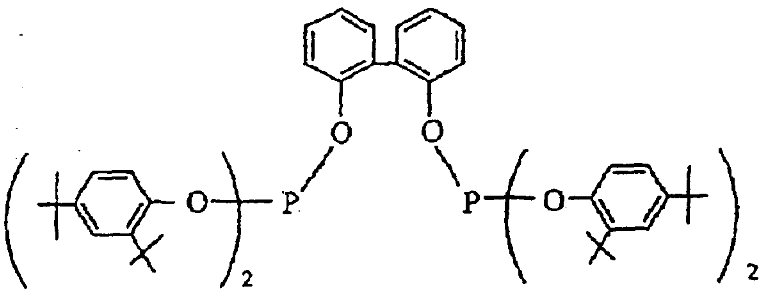

No.12

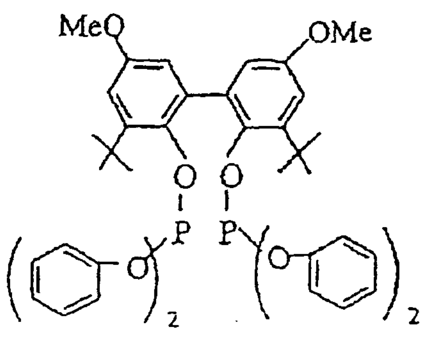

No.13

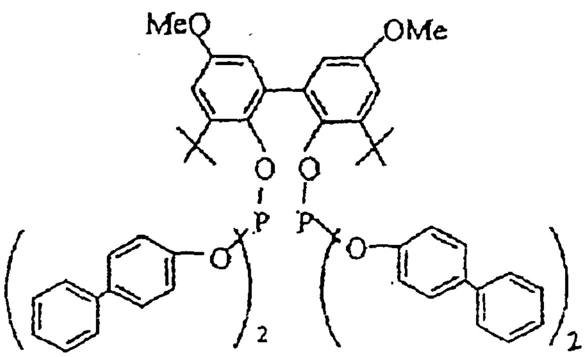

Table 2 (continued)

No.14

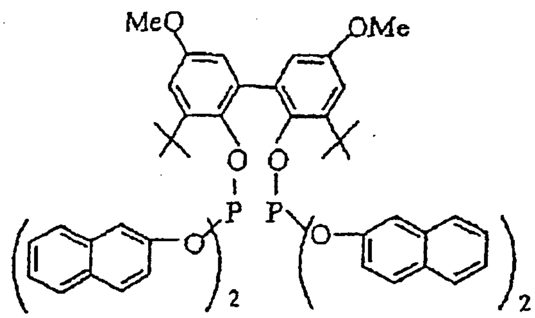

No.15

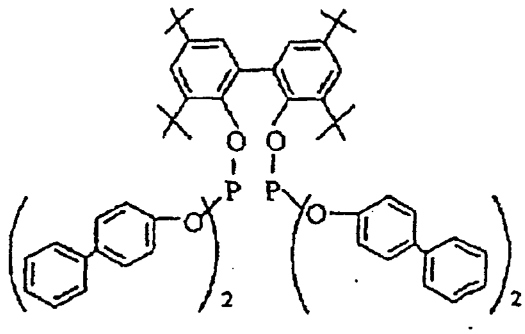

No.16

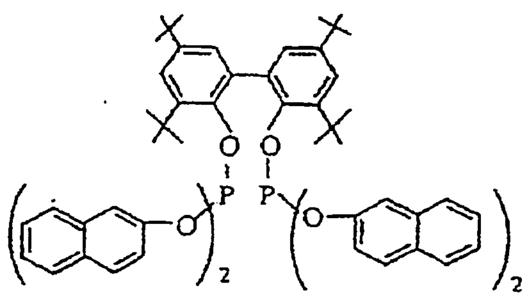

No.17

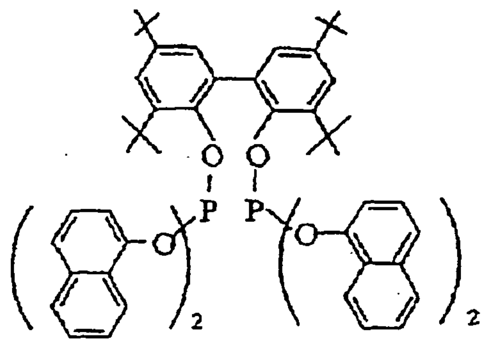

No.18

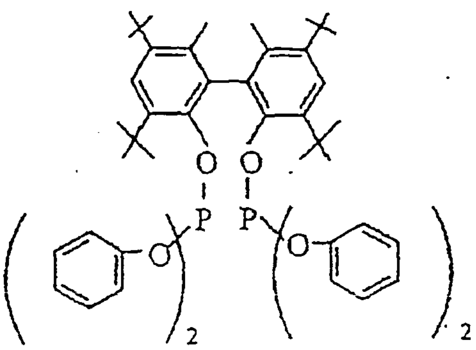

No.19

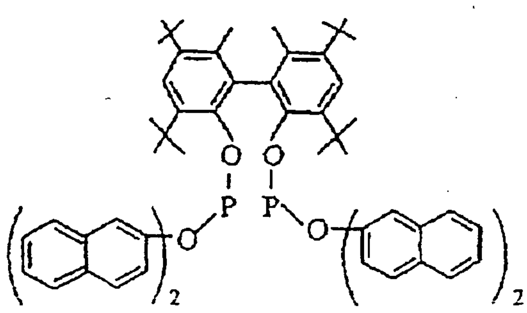

Table 2 (continued)

No.20

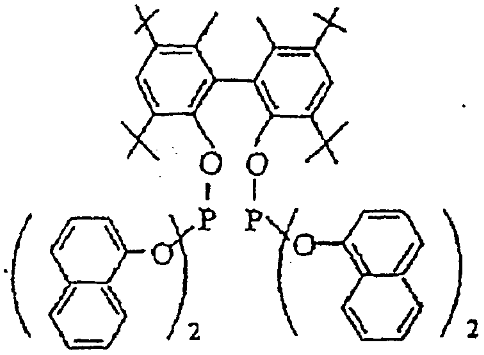

No.21

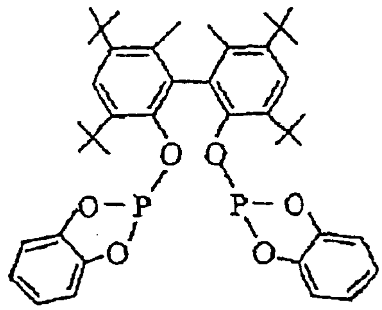

No.22

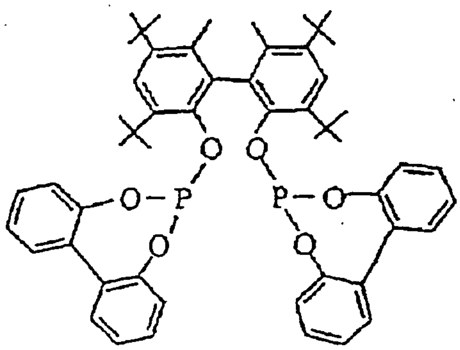

Among the above-mentioned organic phosphites, one having no cyclic structure containing a phosphorus atom, as expressed by the formula (1) is preferable, and a monophosphite is most preferable.

As amines, any one of primary, secondary and tertiary amines can be used, but a tertiary amine is particularly preferable. Particular examples include a substituted or unsubstituted pyridine, quinoline, isoquinoline, indole and phenanthroline, $C_3$-$C_{36}$ trialkylamines, and the like. Also, tertiary polyamines such as poly(2-propenylpyridine), poly(vinylpyridine), and the like can be used.

A concentration of a metal component in a reaction solution is determined depending on a kind of metal used, a reaction substrate and operation conditions of a reactor. For example, when a metal component is rhodium, a metal concentration is usually from 5 ppm to 500 ppm, preferably from 10 ppm to 300 ppm. When the metal component is cobalt, a metal concentration is usually from 50 ppm to 10,000 ppm, preferably from 100 ppm to 5,000 ppm. By feeding a catalyst component at an appropriate place other than an inlet of a continuous multistage reactor, it is possible to operate by varying a catalyst concentration in respective pressure sections.

In the present invention, the reaction may be carried out in the presence of an appropriate solvent if the presence of a solvent is favorable to the process, and also reaction substrates such as an unsaturated hydrocarbon, a product or a high boiling point material by-produced in the reaction system may be used as a solvent without adding a solvent. Such a solvent is preferably inert to the reaction, preferable examples of which include a saturated hydrocarbon compound such as hexane, heptane or the like, and an aromatic hydrocarbon such as toluene, xylene or the like. A compound hardly separatable from a product or a substrate used is not preferable as a solvent to the process.

Also, the reaction can be carried out by adding an appropriate organic or inorganic additive if the addition of

such an additive is favorable to the process.

EXAMPLES

The present invention is further illustrated with reference to Examples, but should not be limited to these Examples.

Reference EXAMPLE 1

As illustrated in the attached Figure 1, a two stage continuous flow reactor having two stirring type autoclave reactors 5 and 9 connected in series was used for reaction. The upper parts of respective reactors had gas-supplying inlets 4 and 10, and the lower parts of the respective reactors had liquid-supplying inlets 3 and 8. A liquid-withdrawing outlet 6 of the reactor 5 and a liquid-withdrawing outlet 11 of the reactor 9 were respectively provided at such positions as to make a liquid phase volume ratio of the reactor 5 and the reactor 9 about 5:2, and a product was withdrawn by overflow from the liquid-withdrawing outlet 11 into a receiver 12.

A mixed octene/m-xylene/pyridine mixture solution (volume ratio 90/5/5) containing 125 mg/ℓ of $Rh(CO)_2(acac)$ was charged into a starting material tank 1, and were continuously fed by means of liquid-feeding pumps 2 and 7 in such a manner as to make an average residence time (total) of the reaction solution in the two flow reactors about 7 hours. The reactors 5 and 9 were heated by heaters from outside, and respective liquid temperatures were controlled to 130°C, and a mixed gas of $H_2/CO$ (ratio 1/1) was continuously supplied to respective reactors from the gas-supplying inlets 4 and 10 so as to make an internal pressure (total pressure of hydrogen and carbon monoxide) in the reactor 5 about 50 kg/cm$^2$ and an internal pressure (total pressure of hydrogen and carbon monoxide) in the reactor 9 about 170 kg/cm$^2$.

The composition of the mixed octene used herein was n-octenes 16%, 3-methylheptenes 66% and 3,4-dimethylhexenes 18%, and was obtained by dimerization of butene.

The reaction was continued until the composition of the liquid withdrawn from the liquid-withdrawing outlet 11 became substantially constant, and as this result, a conversion of the mixed octene was 92.2%, and a C9 alcohol/C9 aldehyde ratio in the product solution was 0.0896, and a selectivity of a terminal formylated product in (C9 alcohol + C9 aldehyde) was 42.8%.

The terminal formylated product selectivity is a ratio of a total amount of (1-nonanal, 6-methyloctanal, 4-methyloctanal, 4,5-dimethylheptanal, 1-nonanol, 6-methyloctanol, 4-methyloctanol and 4,5-dimethylheptanol) to the total amount of (C9 alcohol + C9 aldehyde).

The aldehyde product thus obtained is hydrogenated by a usual method using a catalyst such as nickel/diatomaceous earth, copper chromite or the like to obtain an alcohol suitable for use as a raw material of plasticizer, and the like.

COMPARATIVE EXAMPLE 1

The same procedure as in Reference Example 1 was carried out, except that both internal pressures in the reactor 5 and in the reactor 9 were controlled to 170 kg/cm$^2$ and a total average residence time of the reaction solution was made 3 hours. As this result, a reaction conversion of mixed octene was 92.8%, and a C9 alcohol/C9 aldehyde ratio in the product solution was 0.0512, but a selectivity of a terminal formylated product was only 35.25%.

COMPARATIVE EXAMPLE 2

The same procedure as in Reference Example 1 was carried out, except that both internal pressures in the reactor 5 and in the reactor 9 were controlled to 50 kg/cm$^2$. In order to make a conversion of mixed octene at least 90%, it was necessary to make a total average residence time in the reactors 5 and 9 a long time of about 20 hours, and it was proved that such a long residence time could not be employed for industrial practical use. After the average residence time of 20 hours, a reaction conversion was 90.4%, and a selectivity of a terminal formylated product was 42.6%, and after an average residence time of 26 hours, a reaction conversion was 93.5% and a selectivity of a terminal formylated product was 42.2%.

By employing the process of the present invention, both of a high conversion of olefin and a high terminal formylation rate of a product can be achieved at the same time. Also, since it is possible to increase an amount of an alcohol product produced together with an aldehyde product, the present invention provides a great industrial merit of reducing a load of hydrogenation at a later step when the aldehyde product is used for preparing an alcohol for plasticizer.

## Claims

1. A process for preparing aldehydes which comprises reacting an olefinic; unsaturated compound with hydrogen and carbon monoxide by hydroformylation reaction in the presence of a catalyst in a continuous multistage flow reactor wherein the reaction starting material of liquid phase is transferred from an upper reactor to a lower reactor of the multistage flow reactor, wherein the continuous multistage flow reaction contains n number ($n \geq 2$) of flow reactors, and the reaction is carried out in the presence of pressure sectional zones satisfying the following pressure condition (2), provided that partial pressures of hydrogen in each reactor are illustrated as ($P_{H2}$ (1)), ($P_{H2}$ (2)), ... ($P_{H2}$ (n)) in order from the upper stream from the reactor,

$$(2) \qquad P_{H2}(m-1) < P_{H2}(m)$$

in which m is an integer in the range of $2 \leq m \leq n$ wherein the reaction is carried out in a pressure sectional zone in which B/A is from 1.2 to 10, provided that among hydrogen partial pressures of n number of reactors, the lowest partial pressure value is A and the highest partial pressure value is B.

2. The process according to claim 1, wherein the reaction is carried out in the presence of a pressure sectional zone further satisfying the following pressure condition (1), provided that partial pressures of carbon monoxide in each reactor are illustrated as ($P_{CO}$ (1)), ($P_{CO}$ (2)), ... ($P_{CO}$ (n)) in order from the upper stream from the reactor,

$$(1) \qquad P_{CO}(m-1) < P_{CO}(m)$$

in which m is an integer in the range of $2 \leq m \leq n$.

3. The process according to claim 1, wherein the reaction is carried out in the presence of a pressure sectional zone further satisfying the following pressure condition (3),

$$(3) \qquad P_{CO}(m-1) + P_{H2}(m-1) < P_{CO}(m) + P_{H2}(m)$$

in which m is an integer in the range of $2 \leq m \leq n$.

4. The process according to any one of claims 1 to 3, wherein the olefinic unsaturated compound is an internal olefin.

5. The process according to claim 4, wherein the internal olefin is one component of an octene mixture obtained by dimerization of butene.

6. The process according to any one of claims 1 to 5, wherein the olefinic unsaturated compound is a $C_3$-$C_{12}$ unsaturated compound.

7. The process according to any one of claims 1 to 3, wherein m is an integer of 2 in any one of the above formulae (1) to (3).

8. The process according to any one of claims 1 to 7, wherein the reaction is carried out in a pressure sectional zone in which D/C from 1.8 to 10, provided that among carbon monoxide partial pressures of n number of reactors, the lowest partial pressure value is C and the highest partial pressure value is D.

9. The process according to any one of claims 1 to 8, wherein the reaction is carried out in a pressure sectional zone in which F/E is from 1.1 to 10, provided that among total partial pressures of hydrogen and carbon monoxide in n number of reactors, the lowest total partial pressure value is E and the highest total partial pressure value is F.

10. The process according to any one of claims 1 to 9, wherein the number (n) of continuous flow reactors is from 2 to 5.

**11.** The process according to any one of claims 1 to 10, wherein the catalyst contains an element of Groups 8 to 10 of the Periodic Table,

**12.** The process according to any one of claims 1 to 11, wherein the catalyst contains a ligand selected from the group consisting of phosphines, phosphites and amines.

**13.** The process according to any one of claims 1 to 12, wherein a reaction conversion of the olefinic unsaturated compound is from 75 to 100%.

**14.** A process for preparing alcohols, which comprises preparing aldehydes by any one of the methods according to any one of claims 1 to 13 and then hydrogenating the aldehydes thus prepared.

**Patentansprüche**

**1.** Verfahren zur Herstellung von Aldehyden, umfassend das Umsetzen einer olefinischen ungesättigten Verbindung mit Wasserstoff und Kohlenmonoxid durch Hydroformylierungsreaktion in Gegenwart eines Katalysators in einem kontinuierlichen Mehrstufen-Strömungsreaktor, worin das Reaktionsausgangsmaterial in flüssiger Phase von einem oberen Reaktor zu einem unteren Reaktor in dem Mehrstufenströmungsreaktor geleitet wird, wobei der kontinuierliche Mehrstufen-Strömungsreaktor eine n-Anzahl ($n \geq 2$) an Strömungsreaktoren enthält, und die Umsetzung in Gegenwart von Drucksektionszonen durchgeführt wird, welche der folgenden Druckbedingung (2) genügen, vorausgesetzt, dass die Partialdrucke von Wasserstoff in jedem Reaktor wiedergegeben sind als ($P_{H2}$ (1)), ($P_{H2}$ (2)), ... ($P_{H2}$ (n)), in der Reihenfolge von dem oberen Strom von dem Reaktor,

$$(2)\quad P_{H2}(m-1) < P_{H2}(m)$$

worin m eine ganze Zahl im Bereich von $2 \leq m \leq n$ ist, wobei die Umsetzung in einer Drucksektionszone durchgeführt wird, worin B/A 1,2 bis 10 beträgt, vorausgesetzt, dass unter Wasserstroffpartialdrucken einer n-Anzahl von Reaktoren der niedrigste Partialdruckwert A ist und der höchste Partialdruckwert B ist.

**2.** Verfahren nach Anspruch 1, wobei die Umsetzung in Gegenwart einer Drucksektionszone durchgeführt wird, welche weiterhin der folgenden Druckbedingung (1) genügt, vorausgesetzt, dass die Partialdrucke von Kohlenmonoxid in jedem Reaktor wiedergegeben sind als ($P_{CO}$ (1)), $P_{CO}$ (2)), ... ($P_{CO}$ (n)), in der Reihenfolge von dem oberen Strom von dem Reaktor,

$$(1)\quad P_{CO}(m-1) < P_{CO}(m)$$

worin m eine ganze Zahl im Bereich von $2 \leq m \leq n$ ist.

**3.** Verfahren nach Anspruch 1, wobei die Umsetzung in Gegenwart einer Drucksektionszone durchgeführt wird, welche weiterhin der folgenden Druckbedingung (3) genügt,

$$(3)\qquad P_{CO}(m-1) + P_{H2}(m-1) < P_{CO}(m) + P_{H2}(m)$$

worin m eine ganze Zahl im Bereich von $2 \leq m \leq n$ ist.

**4.** Verfahren nach mindestens einem der Ansprüche 1 bis 3, wobei die olefinische ungesättigte Verbindung ein inneres Olefin ist.

**5.** Verfahren nach Anspruch 4, wobei das innere Olefin eine Komponente einer Octenmischung ist, die durch Dimerisation von Buten erhalten wird.

6. Verfahren nach mindestens einem der Ansprüche 1 bis 5, wobei die olefinische ungesättigte Verbindung eine ungesättigte $C_3$-$C_{12}$-Verbindung ist.

7. Verfahren nach mindestens einem der Ansprüche 1 bis 3, wobei m eine ganze Zahl von 2 in jeder der obigen Formeln (1) bis (3) ist.

8. Verfahren nach mindestens einem der Ansprüche 1 bis 7, wobei die Umsetzung in einer Drucksektionszone durchgeführt wird, worin D/C 1,8 bis 10 beträgt, vorausgesetzt, dass unter Kohlenmonoxidpartialdrucken einer n-Anzahl von Reaktoren der niedrigste Partialdruckwert C ist und der höchste Partialdruckwert D ist.

9. Verfahren nach mindestens einem der Ansprüche 1 bis 8, wobei die Umsetzung in einer Drucksektionszone durchgeführt wird, worin F/E 1,1 bis 10 beträgt, vorausgesetzt, dass unter Gesamtpartialdrucken von Wasserstoff und Kohlenmonoxid in einer n-Anzahl von Reaktoren der niedrigste Gesamtpartialdruckwert E ist und der höchste Gesamtpartialdruckwert F ist.

10. Verfahren nach mindestens einem der Ansprüche 1 bis 9, wobei die Anzahl (n) kontinuierlicher Strömungsreaktoren 2 bis 5 beträgt.

11. Verfahren nach mindestens einem der Ansprüche 1 bis 10, wobei der Katalysator ein Element der Gruppen 8 bis 10 des Periodensystems enthält.

12. Verfahren nach mindestens einem der Ansprüche 1 bis 11, wobei der Katalysator einen Liganden enthält, gewählt aus der Gruppe, bestehend aus Phosphinen, Phosphiten und Aminen.

13. Verfahren nach mindestens einem der Ansprüche 1 bis 12, wobei die Reaktionsumwandlung der olefinischen ungesättigten Verbindung 75 bis 100% beträgt.

14. Verfahren zur Herstellung von Alkoholen, umfassend das Herstellen von Aldehyden nach mindestens einem der Verfahren gemäß mindestens einem der Ansprüche 1 bis 13 und danach das Hydrieren der so hergestellten Aldehyde.

## Revendications

1. Procédé de préparation d'aldéhydes, comprenant la réaction d'un composé à insaturation oléfinique avec de l'hydrogène et du monoxyde de carbone selon une réaction d'hydroformylation en présence d'un catalyseur dans un réacteur à plusieurs étages à circulation, dans lequel le matériau de départ de la réaction de la phase liquide est transféré d'un réacteur supérieur à un réacteur inférieur du réacteur à plusieurs étages à circulation continue, dans lequel le réacteur à plusieurs étages à circulation continue contient un nombre n ($n \geq 2$) de réacteurs à circulation, et la réaction est effectuée en présence de zones à sections de pression satisfaisant à la condition de pression (2) suivante, les pressions partielles d'hydrogène dans chaque réacteur étant représentées par ($P_{H2}$ (1)), ($P_{H2}$ (2)), ... ($P_{H2}$ (n)) dans l'ordre à partir du courant supérieur issu du réacteur :

$$(2)\ P_{H2}\ (m-1) < P_{H2}\ (m)$$

m étant un entier dans la plage de $2 \leq m \leq n$, dans lequel la réaction est effectuée dans une zone à sections de pression dans laquelle B/A est de 1,2 à 10, à condition que parmi les pressions partielles d'hydrogène du nombre n de réacteurs, la valeur de la pression partielle la plus faible soit A et la valeur de la pression partielle la plus élevée soit B.

2. Procédé selon la revendication 1, dans lequel la réaction est effectuée en présence d'une zone à sections de pression satisfaisant en outre à la condition de pression (1) suivante, les pressions partielles de monoxyde de carbone dans chaque réacteur étant représentées par ($P_{CO}$(1)), ($P_{CO}$ (2)), ... ($P_{CO}$ (n)) dans l'ordre à partir du courant supérieur issu du réacteur :

$$(1)\ P_{co}\ (m\text{-}1) < P_{co}\ (m)$$

m étant un entier dans la plage de $2 \leq m \leq n$.

**3.** Procédé selon la revendication 1, dans lequel la réaction est effectuée en présence d'une zone à sections de pression satisfaisant en outre à la condition de pression (3) suivante :

$$(3)\ P_{co}\ (m\text{-}1) + P_{H2}\ (m\text{-}1) < P_{co}\ (m) + P_{H2}\ (m)$$

m étant un entier dans la plage de $2 \leq m \leq n$.

**4.** Procédé selon l'une quelconque des revendications 1 à 3, dans lequel le composé à insaturation oléfinique est une oléfine interne.

**5.** Procédé selon la revendication 4, dans lequel l'oléfine interne est un composant d'un mélange d'octènes obtenu par dimérisation de butène.

**6.** Procédé selon l'une quelconque des revendications 1 à 5, dans lequel le composé à insaturation olénique est un composé insaturé en $C_3$-$C_{12}$.

**7.** Procédé selon l'une quelconque des revendications 1 à 3, dans lequel m est un entier égal à 2 dans n'importe laquelle des formules (1) à (3) ci-dessus.

**8.** Procédé selon l'une quelconque des revendications 1 à 7, dans lequel la réaction est effectuée dans une zone à sections de pression dans laquelle D/C est de 1,8 à 10, à condition que parmi les pressions partielles de monoxyde de carbone du nombre n de réacteurs, la valeur de pression partielle la plus faible soit C et la valeur de la pression partielle la plus élevée soit D.

**9.** Procédé selon l'une quelconque des revendications 1 à 8, dans lequel la réaction est effectuée dans une zone à sections de pression dans laquelle F/E est de 1,1 à 10, à condition que parmi les pressions partielles totales d'hydrogène et de monoxyde de carbone du nombre n de réacteurs, la valeur de la pression partielle totale la plus faible soit E et la valeur de la pression partielle la plus élevée soit F.

**10.** Procédé selon l'une quelconque des revendications 1 à 9, dans lequel le nombre (n) de réacteurs à circulation continue est de 2 à 5.

**11.** Procédé selon l'une quelconque des revendications 1 à 10, dans lequel le catalyseur contient un élément des groupes 8 à 10 du tableau périodique.

**12.** Procédé selon l'une quelconque des revendications 1 à 11, dans lequel le catalyseur contient un ligand choisi parmi le groupe comprenant les phosphines, les phosphites et les amines.

**13.** Procédé selon l'une quelconque des revendications 1 à 12, dans lequel le taux de conversion par réaction du composé à insaturation oléfinique, est de 75 à 100 %.

**14.** Procédé de préparation d'alcools, comprenant la préparation des aldéhydes selon l'un quelconque des procédés de l'une quelconque des revendications 1 à 13, et ensuite l'hydrogénation des aldéhydes ainsi préparés.

FIG. 1

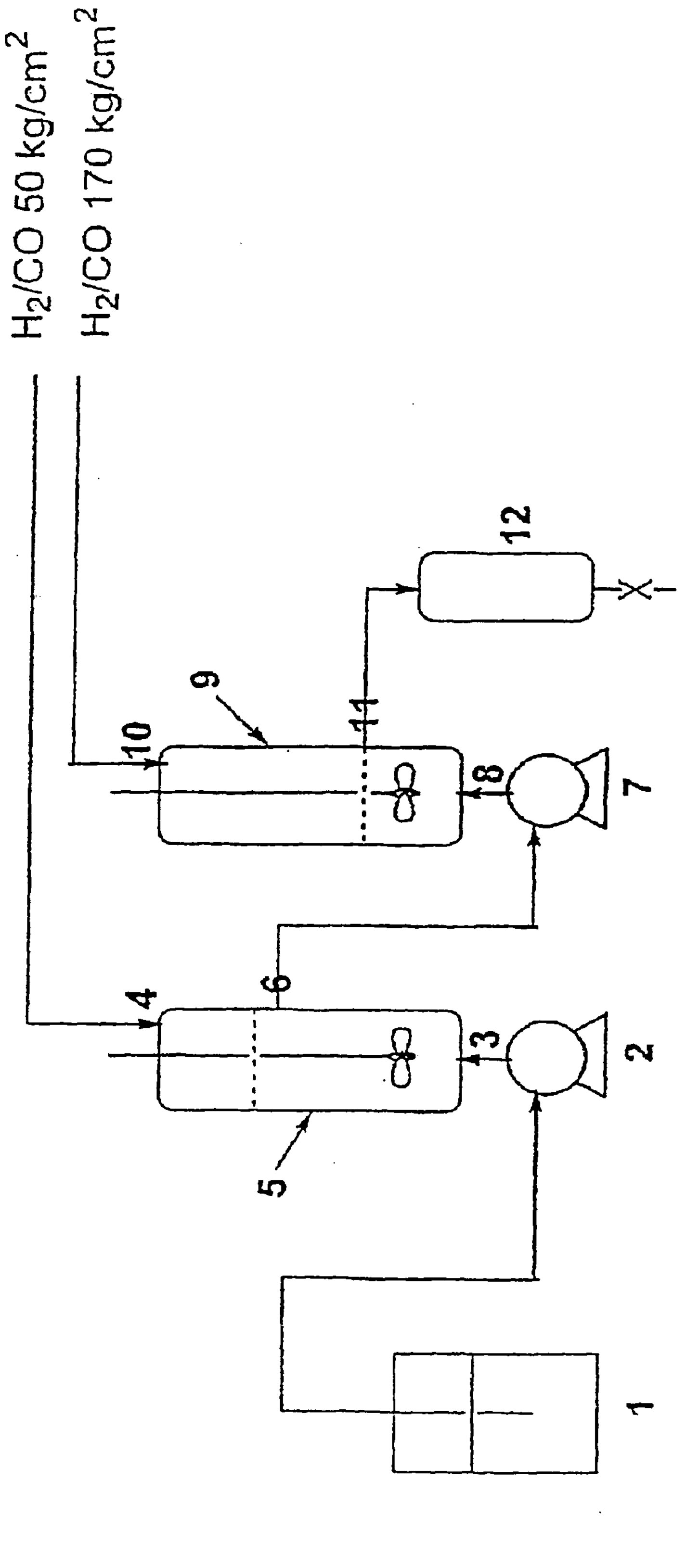

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 61218546 A
- JP 3204831 A
- US 4716250 A